# EUROPEAN PATENT SPECIFICATION

(11) **EP 4 599 118 B1**
(45) Date of publication and mention of the grant of the patent: **01.07.2026**
(21) Application number: 23786812.0
(22) Date of filing: 02.10.2023
(51) Int. Cl.: E03B 1/04, E03C 1/00, G01N 21/25, G01N 21/51, G01N 33/18

(54) **WATER RECYCLING SYSTEM AND METHOD OF RECYCLING WATER IN SUCH A WATER RECYCLING SYSTEM**
WASSERRECYCLINGSYSTEM UND VERFAHREN ZUM RECYCLING VON WASSER IN EINEM SOLCHEN WASSERRECYCLINGSYSTEM
SYSTEME DE RECYCLAGE D'EAU ET PROCEDE DE RECYCLAGE D'EAU DANS UN TEL SYSTEME DE RECYCLAGE D'EAU

(30) Priority: 06.10.2022 SE 2251155
(43) Date of publication of application: 13.08.2025
(73) Proprietor: Mimbly AB, 416 64 Göteborg (SE)
(72) Inventor: VESTMAN, Carl Emil, 41664 Göteborg (SE); KNUTSSON, Jonathan, 416 64 Göteborg (SE)
(74) Representative: Henricson Briggs, David James
(86) International application number: PCT/SE2023/050976
(87) International publication number: WO 2024/076280

(56) References cited:
- EP-A1- 3 187 644
- EP-A1- 3 875 665
- US-A- 3 915 857

## Description

### Field of the Invention

The present disclosure relates to a water recycling system for recycling process wash water, and to a method of recycling water in a water recycling system using such a water recycling system. In particular it relates to a water recycling system comprising a filtration tank, storage tank and manifold.

### Background of the invention

Fresh water is a scarce resource, only between 0.01% to 2.75% of the water on earth is fresh water. Water processing devices, such as laundry machines and dishwashers etc. may use up to 100 L of fresh water in a single washing process. Recycling water used in water processing devices is therefore important.

Water recycling devices receive used process water and retain the water for later use. Such a water recycling device is described in WO 2019/117789 A1. The device of WO 2019/117789 A1 receives water from a water consumption device, and then stores or disposes water based on the measured water quality. The water stored may be distributed to the water consumption device. In the device the filtering, measuring and storage of water occurs a single time.

Filtering particulate debris from process wash water is an especially important aspect of water recycling as debris, such as textile particles, can sully water and lead to reduced recycling performance. Furthermore textiles made of synthetic materials may leech microplastics. Microplastics from synthetic textiles may subsequently enter and pollute waterways and oceans.

US 3 915 857 A (Olson Winston O) discloses an apparatus and process for conserving water in a household comprising cycling "white water" repeatedly through at least one filter until suspended solids and dissolved organic material is removed, and then returning the filtered water to a household system.

EP 3 875 665 A1 (Samsung Electronics Co Ltd) discloses a washing machine that detects contamination of water due to a dye via a light emitting element emitting a plurality of visible rays of different wavelengths, and infrared ray, and a light receiving element.

EP 3 187 644 A1 (Qingdao Haier Washing Mach Co) discloses a washing machine having a colour sensor for detecting the colour of the washing water, and determining if the clothing in the washing machine is fading based on the colour of the washing water.

Improved devices which are capable of receiving and recycling greater volumes of water would be ideal.

### Summary of the invention

The present invention is directed to a water recycling system as defined in appended independent claim 1. The present invention is also directed to a method of recycling water in a water recycling system as defined in appended independent claim 12. The scope of the present invention is solely restricted and defined by the appended claims.

Accordingly, the present invention, defined by the claims, preferably seeks to mitigate, alleviate or eliminate one or more of the above-identified deficiencies in the art and disadvantages singly or in any combination and solves at least the above mentioned problems by providing a water recycling system, amongst other features comprising a filtration tank for receiving and filtering processed wash water. The device comprises a manifold provided with a plurality of inlets for receiving water, a plurality of valves for controlling fluid flow, and a plurality of outlets for distributing water. The device further comprises a storage tank for receiving and storing processed water from the filtration tank via a first outlet of the manifold. At least one inlet of the manifold is provided such that water may be received from the storage tank into the manifold, and wherein the manifold comprises a second outlet to the filtration tank such that water may be circulated through the water recycling device.

A water recycling system, defined by appended claim 1, comprising a water collection unit is provided.

A method of recycling water in the water recycling system, defined by appended claim 12, is also provided.

Further advantageous embodiments are disclosed in the appended and dependent patent claims.

### Brief description of the drawings

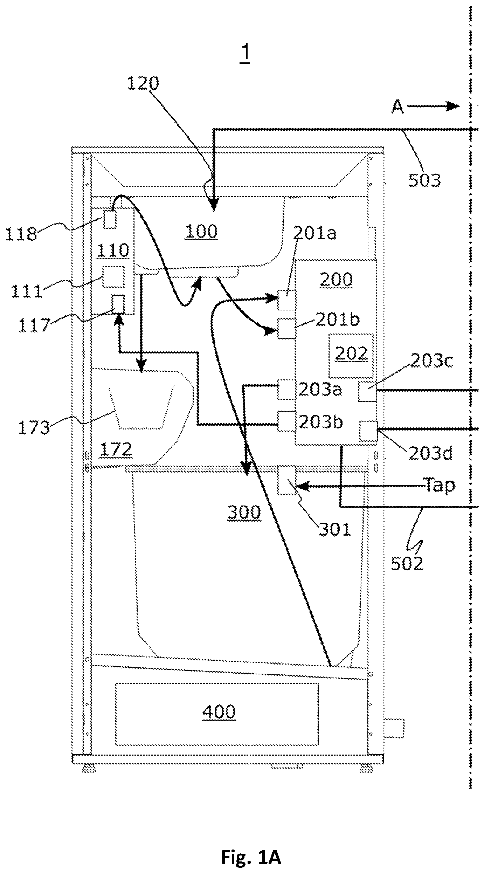

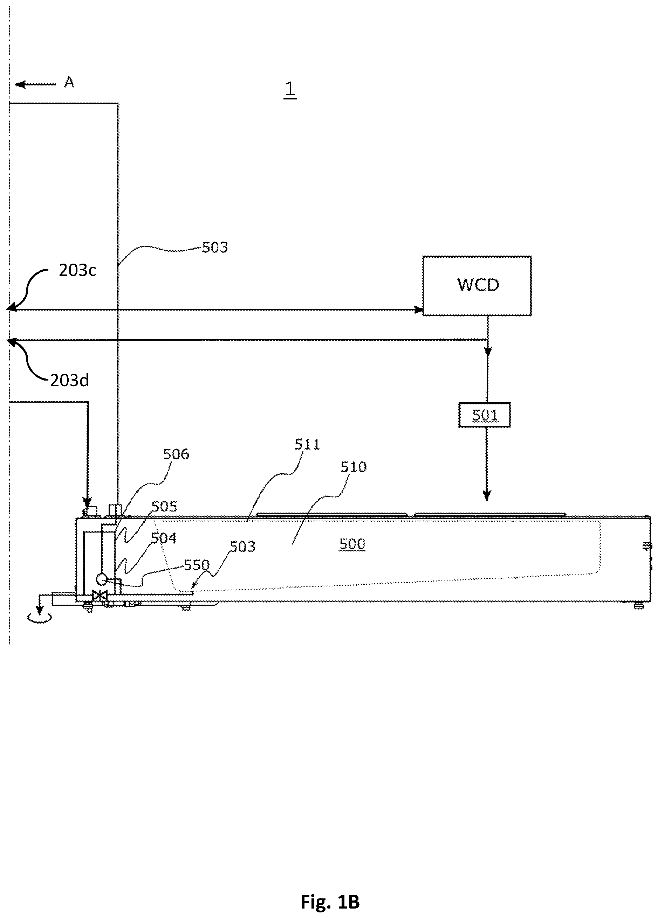

These and other aspects, features and advantages of which the invention, defined by the claims, is capable will be apparent and elucidated from the following description of embodiments of the present invention, reference being made to the accompanying drawings, in which
Figs. 1A and B show a schematic view of a water recycling system comprising a water recycling device and a water collection unit. Figs. 1A and B are separated by the dotted-and-dashed line A which defines the cut point of the two figures.
Fig. 1A is a schematic view of a water recycling device according to an aspect.
Fig. 1B is a schematic view of a water collection unit showing according to an aspect. The WCD being a Water Consumption Device, such as a washing machine and/or dishwasher.
Fig. 2A is a schematic partial cross-section of a filtration tank according to an aspect.
Fig. 2B is a schematic top-down view of a rotatable backflushing arm according to an aspect.
Fig. 3A is a schematic top-down view of a water quality sensing device according to an aspect. The dotted region represents water in the receptacle.
Fig. 3B is a schematic side-view of a water quality sensing device according to an aspect. The dotted region represents water in the receptacle.
Fig. 4 is a schematic partial cross-section of a filtration tank according to an aspect.

### Detailed description

The present disclosure relates to a water recycling system comprising a water recycling device 1. The water recycling device 1 comprises a filtration tank 100 for receiving and filtering processed wash water. The device 1 comprises a manifold 200. The manifold 200 is provided with a plurality of inlets 201, a plurality of valves 202, and a plurality of outlets 203 for distributing water from the manifold 200. The recycling device 1 comprises a storage tank 300 for receiving and storing processed water. The storage tank receives water from at least the filtration tank 100. The water received in the storage tank 300 is received via a first outlet 203a of the manifold 200. The manifold 200 comprises at least one inlet 201a such that water may be received from the storage tank 300 into the manifold 200. The manifold 200 comprises a second outlet 203b in connection with the filtration tank 100, such that water may be circulated through the water recycling device 1. Processed water is receivable in the filtration tank 100, the water is distributed to the storage tank 300, and may be distributed back to the filtration tank 100 from the storage tank 300 via the manifold 200.

The two-way connection of the filtration tank 100 to the storage tank 300 via the manifold 200 enables water to be circulated through the water recycling device 1 such that it may be filtered several times, used to clean the filtration tank 100 and manifold 200, and generally improves filtration, water recycling and water availability performance of the water recycling device 1.

The manifold 200 is a single unit comprising internal channels. The internal channels connect the plurality of inlets 201 to the plurality of valves 202 and outlets 203. The manifold 200 may be a substantially monolithic element in which the internal channels are moulded, drilled etc. By providing the manifold 200 as a single unit maintenance of the water recycling device 1 is eased as inlets 201, valves 202 and outlets 203 are positioned at the same point in the water recycling device 1.

The manifold 200 comprises an outlet 203c which is connected to a water consumption device. The outlet 203c provides water to a water consumption device for recycling. The outlet 203c is ideally in connection with a pressure sensor, temperature sensor and/or a flow meter. The outlet 203c thereby provides water the pressure sensor, temperature sensor and/or flow meter prior to provision to the water consumption device. The pressure/temperature/flow of the water can be controlled such that the water consumption device receives the correct volume of water, and optionally at a temperature suitable for the specific purpose of the water consumption device, for example, a rinse or wash cycle.

The filtration tank 100 comprises a filter 150. The filtration tank 100 of the water recycling system 1 comprises a filter-side portion 101. The filter-side portion 101 refers to the portion of the filtration tank 100 which is within the filter 150. Any water within the filter-side portion 101 has passed through the filter 150 at least once. The filtration tank 100 will be described in further detail below.

The water recycling device 1 may comprise at least one water quality and sensing unit 110. The water quality sensing unit 110 comprises at least one water quality sensor 111. The water quality sensing unit 110 comprises an inlet 117 such that water can be received in the water quality sensing unit 110. The inlet 117 receives water downstream from the filtration tank 100. The water quality sensing unit 110 therefore receives filtered processed water from the filtration tank 100. An outlet 118 of the water quality sensing unit 110 is provided in connection with the filter-side portion 101 of the filtration tank 100. Water flowing from the water quality sensing unit 110 is directed to the filter-side portion 101 of the filtration tank 100. The water quality sensing unit 110 therefore always receives processed water which has been filtered at least one time. The processed and filtered water which has undergone water quality measurement is provided to the filter-side portion 101 of the filtration tank and therefore the quality of the water being used to flush the filter-side portion 101 is measurable and generally known by the water quality recycling system and device 1. The water quality sensing unit 110 is especially advantageous in the present device 1 as water is repeatedly filtered, stored etc. and therefore the water quality could deteriorate over time. By providing the water quality sensing unit 110 water need not be discarded based on a duration of storage or based on the quality of water first received via the water consumption device.

The inlet 117 and outlet 118 may be connected to valves within the manifold 200 such that during a water quality measurement process the flow through the water quality sensing unit 110 is stationary. That is, the water quality sensing unit 110 may be filled with water, and then a valve within the manifold 200 is closed such that the water is maintained within the unit for the duration of a water quality measurement process. This has been shown to increase measurement performance as higher flow rates cause turbulence which can be misinterpreted as turbidity.

The storage tank 300 is connected to the water quality sensing unit 110 via a fluid conduit. As described above, any water flowing from the storage tank 300 to the water quality sensing unit 110 is thereafter received at the filter-side portion 101 of the filtration tank 100. That is, the quality of water in the storage tank 300 may be measured by the water quality sensing unit 110 and thereafter distributed to the filter-side portion 101 of the filtration tank 100.

The water recycling device 1 has an inlet for receiving tap water, that is, non-processed tap water. The non-processed tap water may be received at each of the filtration tank 100, the storage tank 300 and the water quality sensing unit 110. The non-processed tap water is generally and ideally received at only the storage tank 300 via an inlet 301 provided to an upper part of the storage tank 300. The non-processed tap water then falls due to gravity into the storage tank 300. The air-gap between the inlet 301 and water in the storage tank 300 is provided for regulatory purposes. The non-processed tap water may be used for cleaning the respective elements. The non-processed tap water may also be used for increasing the relative quality of the received water previously received to the filtration tank 100 and/or storage tank 300. That is, if the stored processed water is of lower quality, by adding a sufficient amount of clean tap water, the quality can be improved to a level where it is possible to use in a subsequent process.

The filtration tank 100 is formed by a wall 130 forming a receptacle for water. The wall 130 has a cap 131 which encloses the filtration tank 100. The cap 131, or a portion 132 of the cap 131, is advantageously removable to enable access to the internal region of the filtration tank 100 for maintenance and testing. As will be described below, the cap 131 may be provided in fixed connection to the filter 150 which provides additionally advantages.

As described above, the filtration tank 100 comprises a filter 150. The filter 150 is generally a filter 150 comprising a circumferential screen 151 and a rotatable backflushing arm 152 for providing a stream of backflushing water at the filter side portion 101 of the circumferential screen 151. The circumferential screen 151 may be a substantially cylindrical screen 151, extending within a lower portion of the filtration tank 100 such that it is within any water present in the filtration tank 100. As described previously, the filtration tank 100 comprises a filter-side portion 101. The filtration tank 100 also comprises a non-filter-side portion 102. The non-filter side portion 102 refers to the portion of the filtration tank 100 which comprises water which has been received into the filtration tank 100 but has not yet passed through the filter 150. As process water may pass through the filtration tank 100 several times, when referring to water that has not yet passed through the filter it is intended to mean has not passed through the filter during an instant filtering process, it may have passed previously, i.e., the process water may have previously passed through the filter 150. The filter side portion 101 may be referred to simply as the internal region 101. The non-filter side portion 102 may be referred to simply as the external region 102.

To enable the filtering of microplastics, the screen has perforations having a diameter of less than about 100 µm.

Process water is received in the filtration tank via at least the process inlet 120. The process inlet 120 is arranged in the non-filter-side portion 102 of the filtration tank 100. The process inlet 120 may advantageously be directed directly downwards as this has been shown to increase agitation of water within the filtration tank 100 and improves filtration and backflushing performance. A pressure differential between the filter-side portion 101 and the non-filter side portion 102 causes the water to pass through the filter 150. The pressure differential may be provided by the relative levels of water in the respective portions 101, 102 or may be provided by a pump. The filtration tank 100 comprises an outlet 153 for receiving filtered water from the filter-side portion 101. The filtration tank 100 comprises an inlet 154 for providing water to the filter-side portion 101. The outlet 153 is connected to the manifold 200. The inlet 154 is arranged in fluid connection to the rotatable arm 152 such that water received at the inlet 154 may flow through a channel or cavity in the arm 152 and subsequently be directed towards the internal side of the filter 150 via a radial hole 155 provided to the arm 152.

The circumferential filter screen 151 is maintained in position in the filtration tank 100 via a housing 156. The housing 156 is a substantially cylindrical casing with open portions 157 exposing the filter 150. The circumferential screen 151 may be a cylindrical screen 151, as the housing 156 defines the shape of the screen.

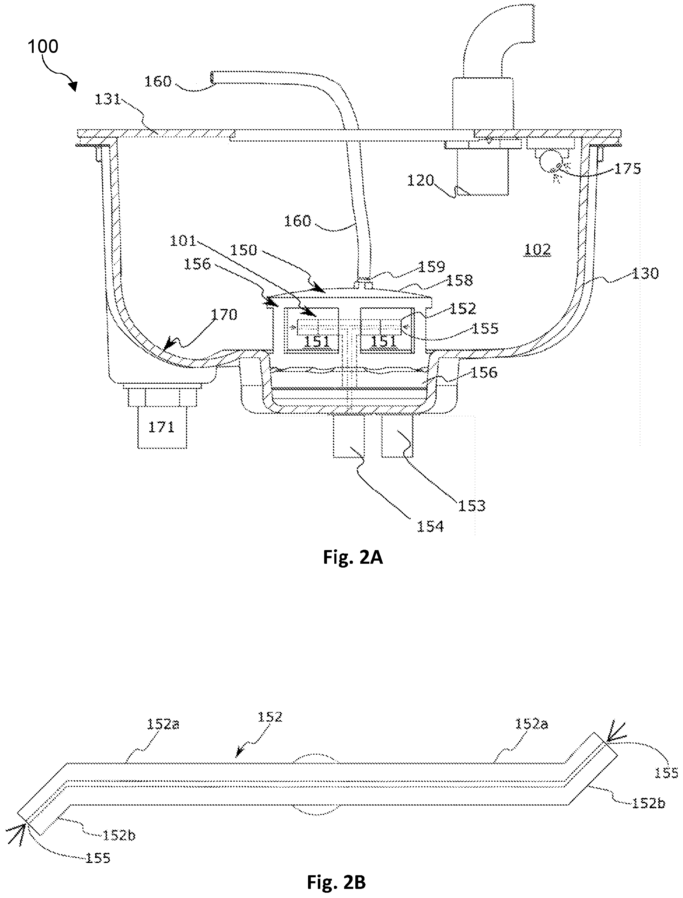

The filter 150 is in a fixed position within the filtration tank 100 during a filtering process. As stated above, the filter 150 is ideally in a lower portion of the filtration tank 100 such that is more likely to be, at least partially, submerged during a filtering process. The filter 150 may be located at the base of the filtration tank 100 as shown in figure 2A.

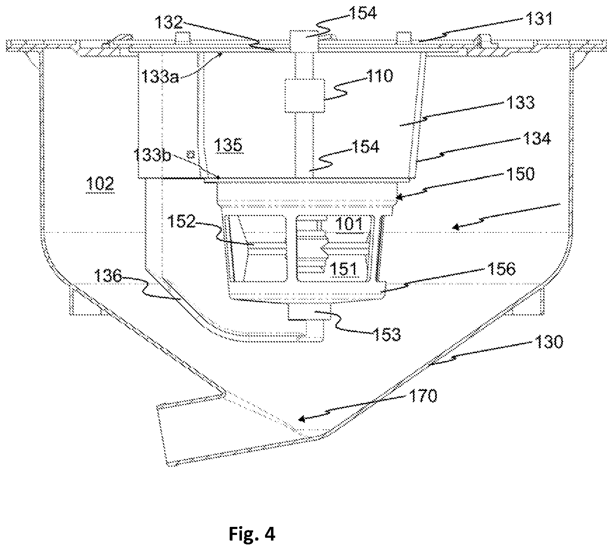

However, the filter 150 need not be fixed to the base of the filtration tank 100. As shown in figure 4, the filter 150, and in particular the housing 156 may be provided in fixed connection to the cap 131, or the portion 132 of the cap 131. In such an arrangement, an extension element 133 is fixed to the cap 131 at a proximal portion 133a, and fixed to the filter 150, e.g., the housing 156, at a distal portion 133b. The extension element 133 defines the location of the filter 150 within the filtration tank 100. The extension element 133 has a solid wall 134 for restricting the ingress of non-filtered process water. The extension element 133 has a substantially hollow inner region 135 such that filtered water may enter the hollow inner region 135 via the filter 150. By providing the filter 150 and extension element 133, the inlet 154 to the rotatable backflushing arm may be provided at the cap 131 and not at the base of the filtration tank 100 as shown in figure 2A. The outlet 153 from the filter-side portion 101 is provided at a lower portion of the filter 150. Advantageously, the outlet 153 may be provided in connection with a fluid conduit 136 which is in a fixed relationship with the extension element 133. In such a manner, removing the cap 131 of the filtration tank 100 removes the extension element 133, filter 150, fluid conduit 136, and the inlet 154 in one piece. This has been shown to reduce maintenance times when inspecting the filter 150. Advantageously, due to the hollow inner portion 135 of the extension element 133, the water quality sensing unit 110 may be provided within the extension element 133 in line with the inlet 154. This reduces errors as it ensures that the water quality measured by the water quality sensing unit 110 is near the backflushing arm 152. Furthermore, by providing the water quality sensing unit 110 within the extension element 133, the water quality sensing unit 110 can be removed in a single step via removing the cap 131, easing maintenance and inspection.

To ensure that air may exit the filter 150 an air outlet 159 is provided to the filter 150. Air present in the filter has been shown to degrade backflushing and therein filtering performance. The air outlet 159 may be formed by the extension 133, and in particular the hollow inner region 135 of the extension. Alternatively, if the housing 156 is provided to the base of the filtration tank 100, the outlet may be provided in an upper wall 158 of the housing 156. The air outlet 159 is connected to a conduit 160 allowing the escape of air above the level of water in the filtration tank 100.

The rotatable arm 152 rotates with respect to the fixed circumferential screen 151. The rotatable arm 152 is activated by the water provided via the inlet 154. The rotatable arm 152 is rotated by the pressure of water flowing through the arm 152 toward the screen 151. The backflushing arm 152 comprises a first portion 152a extends radially outward from the rotational axis of the arm 152 toward the filter screen 151. The backflushing arm comprises a second portion 152b, comprising the hole 155, the second portion 152b extending at an angle from the first portion 152a such that the arm 152 is encouraged to rotate when provided with water. The rotatable arm 152 is not mechanically driven by other means. The flow of water rotates the arm 152 with respect to the screen 151. Water expelled via the hole 155 applies a backflushing force to the screen 151 such that residue is expelled from the screen 151. The backflushing limits clogging of the screen 151 and residue present on the screen 151 is forced into the non-filter side portion 102 of the filtration tank 100. Such backflushing is especially advantageous in process water from washing machines comprises textile particles. This is due to the fact that the textile particles are relatively light weight when compared to e.g., dirt and are easily forced from the screen 151 under backflushing conditions. Previous filter systems such as that in WO 2021/066716 A1 require mechanical scraping of the filter surface to remove particles. The filter 150 of the present recycling system and device 1 does not require mechanical scraping.

Water provided to the filter-side portion 101 of the filtration tank 100 passes the water quality measurement device 110. The water from the outlet 118 of the water quality measurement device 110 is in connection with the inlet 154 of the filtration tank 100. Water passed from the water quality measurement unit 110 is directed to the rotatable backflushing arm 152. As described previously, this leads to the water quality of the backflushing water to be measurable and improves backflushing performance.

The water supplied to the inlet 154 may be provided by a pump 400. The water is subsequently displaced via the pump 400 through the manifold 200, to the inlet 154. The amount of water providable during a backflushing operation is controllable based on the measured water quality. The amount of water may refer to the duration of water provided during a backflushing process, that is, the length of the backflushing process. The amount of water may also refer to the pressure of water provided by the pump 400. The duration and/or pressure of the pump 400 may be controlled depending on the quality of water measured by the water quality sensing unit 110. If the measured water quality by the water quality sensing unit 110 is below a threshold value, the pump 400 may be controlled to pump water at a higher pressure in order to increase the displacement of particles from the filter screen 151. Similarly, the duration of the backflushing process may be controlled in order to increase the displacement of particles from the filter screen 151. By controlling the pump 400 based on the measured water quality, filter clogging can be predicted and avoided before any changes in backpressure or flow via the hole 155 are detected.

In some instances, the water supplied to the inlet 154 may be provided via a clean, tap-water, source operating at mains pressure. The inlet 154 may periodically be provided with tap-water to provide additional flushing to the filter 150. Ideally, the inlet 154 is provided with water from the storage tank 300 via the manifold 200, which may comprise non-processed tap water.

As described previously, during a filter process, the process water passes through the filter screen 151 and exits the filter tank 100 via the outlet 153. The residue is therefore substantially comprised in the non-filter side portion of the tank 102. The residue may exist as a mix of particles and a small volume of water at the base of the tank 100. The tank 100 is provided with a waste outlet 170, connected to a valve 171 which is connected to a residue capture receptacle 172. The residue can therefore be disposed of via the waste outlet 170 to the residue capture receptacle 172. As shown in figure 4, the filtration tank 100 may be provided with a conical bottom portion forming the waste outlet 170. In such a manner filtered waste is captured in the bottom conical portion waste outlet 170.

The residue capture receptacle 172 is a removable container for receiving residue from the filter tank 100. The residue receptacle 172 comprises a filter 173 for filtering any of the residue received in the receptacle 172 from any water in which the residue is comprised. The filter 173 comprises a porous material having pores of less than about 100 µm, such as less than about 85 µm, such as from about 30 µm to about 60 µm. The porous material may have an air permeability of about 400 l/m²/s to about 2500 l/m²/s, such as about 1400 l/m²/s. Such porous materials having the detailed permeability capture the residue received in the capture receptacle 172 and do not retain substantial amounts of water. Ideally, the porous material is cellulose based. The residue capture receptacle 172 is provided directly beneath the waste outlet 170 such that filtrate drops due to the force of gravity into the receptacle 172. Ideally, the residue capture receptacle 172 is user accessible such that it is provided in an opening on an external face of the device 1. The residue receptacle 172 may be provided with a handle at an outer portion such that the residue receptacle 172 may be removed by a single hand of a user.

The filtration tank 100 may be provided with a spray head 175. The spray head 175 provides water at relatively high pressures to the filtration tank 100, and, in particular the non-filter side portion 102 of the filtration tank 100. The spray head 175 is advantageously directed toward the internal face of the wall 130 of the filtration tank 100. The spray head 175 thus directed generates a flow of water starting at the internal face of the wall 130 of the filtration tank 100, the water flows past the non-filter side portion 102 of the filter screen 151 and agitates and removes residue from the base of the filtration tank 100. The residue is thereafter drained via the waste outlet 170. The spray head 175 directed at the wall 130, and not directly at the filter 150, has been shown to combine advantageously with the backflushing process to remove residue, and in particular textile fibres from the filter screen 151. Advantageously, the spray head 175 is activated momentarily after a backflushing process, such that at least a portion of the residue has been removed from the screen 151. The spray head 175 may receive water from the storage tank 300, and/or tap-water at mains pressure.

The water quality sensing device 111 comprises a white light emitting element 112a, an infra-red light emitting element 113a, and a light receiving element 114a. The light receiving element 114a is arranged to receive light from the white light emitting element 112a, and the infra-red light emitting element 113a. The received light at the light receiving element 114a is analysed to determine the turbidity and colour of the filtered process wash water.

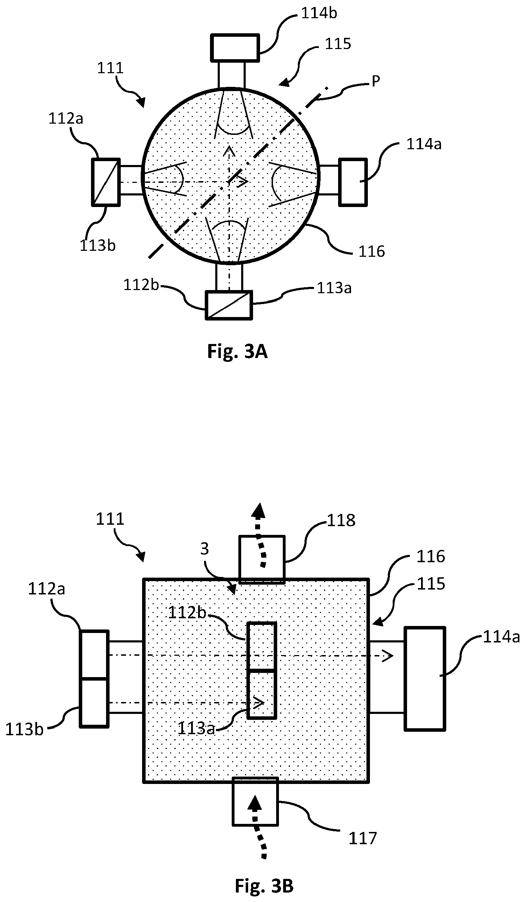

The white light emitting element 112a is arranged co-axial and opposite the light receiving element 114a. The white light emitting element 112a emits light toward the light receiving element 114a. The light emitted from the white light emitting element 112a travels in a substantially straight path to the light receiving element 114a. No reflectors or mirrors are provided to the device 111. The emission axis of the infra-red light emitting element 113a is substantially orthogonal the incident axis of the light receiving element 114a. The path of undisturbed or un-scattered light emitted from the white light emitting element 112a and the infra-red light emitting element 113a are shown in figure 3a by the dashed lines with arrows indicating the direction of light travel.

The emission axis of the infra-red light emitting element 113a is arranged substantially orthogonal the incident axis of the light receiving element 114a. The infra-red light emitting element 113a is not co-axial to the light receiving element 114a. The term substantially orthogonal as used herein means that the angle between the optical incident axis of the light receiving element 114a and the optical emission axis of the infra-red light emitting element 113a is from about 85° to about 95°, such as about 90°. The infra-red light emitting element 113a emits infra-red light in a direction substantially orthogonal to the axis formed between the white light emitting element 112a and the light receiving element 114a. In such an arrangement only infra-red light scattered, absorbed or otherwise disturbed after leaving the infra-red light emitting element 113a is received at the light receiving element 114a. If there is no disturbance, absorbance or scattering of the light emitted from the infra-red light emitting element 113a no, or very little infra-red light is received at the light receiving element 114a.

As shown in figure 3a, and is known within the field, each of the white light emitting element 112a and infra-red light emitting element 113a emit a cone of light, and therefore the optical emission axis is the centre axis of the emitted cone of light. Similarly, the light receiving element 114a has a viewing angle and receives incident light at an angle greater than 0° with respect to the central incident axis. The term light incident axis as used herein refers to the central incident axis.

The white light emitting element 112a ideally emits light across the wavelength spectrum from about 400 nm to about 750 nm. The infra-red light emitting element 113a may emit infra-red light in the infra-red spectrum from about 700 nm to about 1 mm, such as about 750 nm to about 900 nm.

The light receiving element 114a may be an ambient light sensor configured to receive and detect infra-red, red, green and blue light. Ideally, the ambient light sensor is configured to detect red, green, blue and white light, where white light corresponds to broad spectrum detection over at least infra-red, red, green and blue wavelength spectrums.

Both the white light emitting element 112a and the infra-red light emitting element 113a may emit light into a volume of water, such as wash water in order to detect the quality of the water.

The white light emitting element 112a, infra-red light emitting element 113a and the light receiving element 114a may be in some instances arranged in the same plane. In figure 3b the plane is a horizontal plane, however, the plane need not be horizontal. The plane referred to is the plane formed by the optical axes of the white light emitting element 112a, infra-red light emitting element 113a, and the theoretical incident axis of the light receiving element 114a.

The white light emitted from the white light emitting element 112a traverses the volume of water and is received at the light receiving element 114a. The white light emitted from the white light emitting element 112a has a substantially known wavelength spectrum and known intensity over the known spectrum. As the white light emitted from the white light emitting element 112a traverses the water volume the light is substantially filtered and the spectrum and intensity over the spectrum of the emitted white light is altered such that the received light at the light receiving element 114a has different intensities at different wavelengths compared to the white light emitted from the white light emitting element 112a. The altered spectrum of white light received at the light receiving element 114a may be compared to the known emitted spectrum to determine water quality. For example, the colour of the water volume may be determined by comparing the wavelength spectrum, and intensity at various wavelengths of the received light at the light receiving element 114a to the emitted white light from the white light emitting element 112a. If the volume of water has a certain colour this can be determined by comparing the relative intensities of the colours received at the light receiving element 114a. For example, if the water is coloured red, due to for example a dye in the water, the red-coloured water will absorb light in the blue and/or green spectrum, leading to relatively lower intensities of blue and/or green light received at the light receiving element 114a. Similarly, green coloured water results in a reduction of the blue spectrum received at the light receiving element 114a.

The infra-red light emitting element 113a emits infra-red light into the volume of water to determine water quality. Infra-red light emitted from the infra-red light emitting element 113a traverses the volume of water and is at least partially scattered by the water, orthogonal to the light receiving element 114a. A portion of the scattered light is received at the light receiving element 114. The portion of light received at the light receiving element 114a is correlated to the amount of scattering due to the water. Water having a higher turbidity scatters more light, and therefore the portion of light received at the light receiving element 114a corresponds to the level of turbidity of the water. That is, the more light received at the light receiving element 114a from the infra-red light emitting element 113a, the greater the turbidity of the water.

The water quality sensing unit 110, and in particular the water quality sensing device 111, may comprise a receptacle 115 for receiving a volume of fluid, such as wash water. The receptacle 115 receives a volume of water therein for a duration. The receptacle may be defined by at least one wall 116. The receptacle may be a cylinder, or tube as shown in figure 3a. The white light emitting element 112a, infra-red light emitting element 113a and the light receiving element 114a are directed into the receptacle 115. The light emitted from the white light emitting element 112a is directed into the receptacle 115. The light emitted from the infra-red light emitting element 113a is directed into the receptacle 115. The light receiving element 114a has a sensor region which is directed into the receptacle. The light receiving element 114a receives light which has been emitted into the receptacle. When fluid is provided to the receptacle 115, the light from the white light emitting element 112a and/or the infra-red light emitting element 113a is emitted into the volume of fluid. When fluid is provided to the receptacle 115, the light receiving element 114a receives light which has passed through the fluid.

As stated above the path white light travels from the white light emitting element 112a to the light receiving element 114a is substantially straight and free from mirrors or reflecting elements. As would be understood by the skilled person, some minor refraction to the light may occur due to the volume of water, and the wall 116 of the receptacle 115.

The white light emitting element 112a, infra-red light emitting element 113a and the light receiving element 114a are provided external to the receptacle 115. That is, they are not provided within the receptacle and do not mechanically disturb the fluid within the receptacle 115. The receptacle 115 may be provided with a flat, or smooth, internal face without protrusions. The white light emitting element 112a, infra-red light emitting element 113a and light receiving element 114a may be separated from the volume of fluid within the receptacle 115 by the at least one wall 116. By providing the light emitting elements 112a, 113b and the light receiving element 114a separate and external to the volume of fluid the white light emitting element 112a, the infra-red light emitting element 113a and the light receiving element 114a are not subject to fouling, wear or other similar disturbances which may reduce the performance of the device 111. Additionally, by providing the white light emitting element 112a, infra-red light emitting element 113a and the light receiving element 114a external to the water receptacle 115, the white light emitting element 112a, infra-red light emitting element 113a and the light receiving element 114a are protected from damage due to contact with high temperature water. The white light emitting element 112a, infra-red emitting element 113a and light receiving element 114a may be placed directly on an external region of the wall 116, i.e., on the outer surface of the wall 116. As shown in figures 3a and 3b, the white light emitting element 112a, infra-red emitting element 113a and light receiving element 114a may be placed at a distance from the outer surface of the wall 116 and provided with channels through white light may travel. The channels are opaque such that light is contained within the channels, and shielded from the ingress of ambient light.

As shown in figure 3a, the water quality sensing device 111 may comprise two distinct set of white light emitting elements 112a, 112b, infra-red light emitting elements 113a, 113b, and light receiving elements 114a, 114b. The sets are arranged such that they are symmetrical with respect to the axis P shown in figure 3a. As shown in figure 3b, in an arrangement with two distinct sets of emitting and receiving elements, the second white light emitting element 112b is arranged such that its emission axis is parallel to and aligned with the axis of the first infra-red emitting element 113a, and the second infra-red emitting element 113b is arranged such that its emission axis is parallel to and aligned with the axis of the first white light emitting element 112a. The second white light emitting element 112b may be arranged above or below the first infra-red emitting element 113a. The second infra-red emitting element 113b may be arranged above or below the first white light emitting element 113a. the provision of two distinct sets of emitting and receiving elements improves redundancy of the water quality sensing device 111 which is especially relevant in turbidity measurement of process wash water as the receptacle 115 may over-time become fouled such that the emission of light is obscured by particles or build-up on the wall 116 of the receptacle 115.

The water recycling system comprises a water collection unit 500. The water collection unit 500 is arranged to receive processed wash water from a water consumption device, such as a washing machine, dishwasher etc. A water consumption device, as referred to herein, refers to a device which generates process wash water, process wash water may be referred to as grey water, or simply wash water. The process wash water may comprise various cleaning agents, soaps etc. that have been used in the process. As described above, process wash water may comprise particulate from clothing such as textile fibres, microplastics, and dirt. The water collection unit 500 is provided with a first inlet 501 for the provision of process wash water. The water collection unit is provided with a second inlet 502, generally separate to the first inlet 501. The second inlet 502 is for the provision of filtered processed wash water from the water recycling device 1. The water collection unit 500 receives filtered processed wash water from the water recycling device 1, and process wash from the water consumption device. The water collection unit 500 comprises an outlet 503 for providing water in the water collection to the water recycling device 1. The water collection unit 500 is therefore capable of distributing both processed wash water, and filtered processed wash water from the water recycling device 1, to the water recycling device 1.

The manifold 200 comprises an outlet 203d which is connected to the inlet 501 of the water collection unit 500. With such a connection water can be transferred from the water recycling device 1 to the water collection unit 500.

The water collection unit 500 is provided with an overflow outlet 504. The overflow outlet 504 is in fluid connection to and downstream of the outlet 503. The overflow outlet 504 is provided with a siphon 505. The siphon 505 has a crest height 506 below an upper edge 511 of a sidewall 510 of the water collection unit 500. The sidewall 510 defining the volume of water receivable in the water collection unit 500. The siphon 505 acts such that if the water level in the water collection unit 500 exceeds the crest height 506 of the siphon water flow through the siphon to the overflow outlet 504. The siphon ensures that overflow events in the water collection unit 500 are avoided. This is especially relevant as large volumes of water may be provided, almost instantaneously to the water collection unit via the water consumption device. An advantage of having the overflow outlet 504 in fluid connection and downstream of the first outlet 503 is that additional tubing and outlets at various heights, provided to the sidewall 510 of the water collection unit 500 are unnecessary. Furthermore, fluid conduits may be substantially comprised within a single region of the water collection unit 500.

The water collection unit 500 may be provided with a pump 550. The pump 550 is normally configured to provide water from the water collection unit 500 to the water recycling device 1. The pump 550 is connected to the outlet 503. The pump 550 is generally connected to the outlet 503 at a point upstream of the siphon 505.

Water may be continuously cycled from the water recycling device 1 to the separate water collection unit 500, and back again. This has the advantage of enabling multiple passes of treatment and filtering of the water by the water recycling device 1, improving the recycling performance of the water recycling device 1, and ensuring that water does not stagnate within the system.

A method for filtering process wash water received from a water consumption device in the filtration tank 100 will now be described. The method comprises: providing process wash water in the filtration tank 100 via the process inlet 120. After the provision of water, the water is filtered via the filter 150. Filtered process wash water is received in the filter-side portion 101 of the filtration tank 100. Filtered process wash water is received at the outlet 153 at the filter-side portion 101 of the filtration tank 100. Water is provided to the rotatable backflushing arm 152 via the inlet 154, to backflush residue from the non-filter side facing portion 102 of the filter screen 151. The method may also comprise an intermittent cleaning process, the cleaning process comprising: pausing the provision of process wash water in the filtration tank 100 such that the water level in the filtration tank 100 is reduced as water is received and drains via the outlet 153. After the level of water within the filtration tank 100 has reduced, a backflushing process as described above is performed. Thereafter, a momentary spray of water to the spray head 175 may be provided to the filtration tank 100, such that backflushed residue is agitated from the non-filter side portion 102 of the screen 151. The residue and is received at the waste outlet 170. During a filtration process the rate of inflow at the process inlet 120 may be substantially matched to the rate of outflow via the outlet 153. When the recycling device 1 is receiving water from the water collection unit 500 the pump flow rate from the water collection unit 500 may be matched to the pump flow rate from the outlet 153 of the filtration tank 100.

The pump 400 of the water recycling device 1 is arranged to pump water throughout the device 1. The combination of the pump 400 and the manifold 200 means that water may be directed throughout the water recycling device 1, such as to the process inlet 120, to the water quality sensing unit 100, to the water storage tank 300 and to the water collection unit 500 may be achieved by a pump in connection to the manifold 200. Two or more pumps may be connected in parallel to increase pump pressure. Separate pumps are not required for each water transport path within the device 1.

The pump 400, valves 201, valve 171, and water quality sensing device 111, are connected to at least one controller which controls the flow of water within the water recycling device and water recycling system. The controller may be connected to additional sensors such as water level sensors provided to at least the filtration tank 100 and water storage tank 300. Additionally, the controller may be adapted to determine if the water consumption device is in need of water via a flow rate and/or pressure sensor connected to the inlet of the water consumption device. The controller may also be in connection with the water collection unit 500, and in particular valves and pumps therein. The water collection unit 500 may comprise a controller separate but in communication with the controller provided to the water recycling device 1.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Furthermore, although individually listed, a plurality of means, elements or method steps may be implemented by e.g. a single unit, control unit or processor. In addition, singular references do not exclude a plurality. The terms "a", "an", "first", "second" etc. do not preclude a plurality. Reference signs in the claims are provided merely as a clarifying example and shall not be construed as limiting the scope of the claims in any way.

## Claims

1. A water recycling system comprising a water recycling device (1), the water recycling device (1) comprising:
- a filtration tank (100) for receiving and filtering processed wash water,
- a manifold (200) provided with a plurality of inlets (201) for receiving water, a plurality of valves (202), and a plurality of outlets (203) for distributing water,
- a storage tank (300) for receiving and storing filtered processed wash water from the filtration tank (100) via a first outlet (203a) of the manifold (200), wherein, at least one inlet (201a) of the manifold (200) is provided such that water may be received from the storage tank (300) into the manifold (200), and wherein the manifold (200) comprises a second outlet (203b) to the filtration tank (100), such that a two-way connection between the filtration tank (100) and the storage tank (300) via the manifold (200) is provided, such that the processed wash water may be circulated through the water recycling device (1), and such that it may be filtered several times, wherein the water recycling system comprises a separate water collection unit (500), the water collection unit (500) arranged to receive processed wash water from a water consumption device (WCD) via a first inlet (501), and wherein the water collection unit (500) is provided with a separate inlet (502) for receiving filtered processed wash water from the water recycling device (1), and an outlet (503) for providing water from the water collection unit (500) to the water recycling device (1) such that both processed wash water received from a water consumption device (WCD) and processed filtered wash water from the water recycling device (1) may be distributed from the water collection unit (500), via the outlet (503), to the water recycling device (1).

2. The water recycling system according to claim 1, wherein the manifold (200) of the water recycling device (1) comprises an outlet (203c) for the output of processed and filtered water to a water consumption device (WCD).

3. The water recycling system according to claim 1 or 2, wherein the water
recycling device (1) comprises a pump (400) in connection with the manifold (200) for the distribution of water.

4. The water recycling system according to any of claims 1 to 3, wherein the
manifold (200) of the water recycling device (1) is a single unit comprising internal channels connecting the plurality of inlets (201) to the plurality of valves (202) and outlets (203).

5. The water recycling system according to any of claims 1 to 4, wherein the water
recycling device (1) comprises a water quality sensing unit (110) comprising at least one water quality sensing device (111), and wherein an inlet (117) to the water quality sensing unit (110) is arranged downstream of the filtration tank (100) such that the water quality sensing unit (110) receives filtered processed wash water, and
wherein an outlet (118) of the water quality sensing unit (110) is arranged to provide water to a filter-side portion (101) of the filtration tank (100).

6. The water recycling system according to claim 5, wherein the water recycling
device (1) is configured to direct the water in the storage tank (300) to the water quality sensing unit (110), and thereafter to the filter-side portion (101) of the filtration tank (100).

7. The water recycling system according to any of claims 5 or 6, wherein the water
recycling device (1) is configured to provide non-processed tap water to the storage tank (300) via an inlet (301) at an upper portion of the storage tank (300).

8. The water recycling system according to any of claims 5 to 7, wherein the water quality sensing device (111) comprises a white light emitting element (112a) configured to emit substantially white light, an infra-red light emitting element (113a) configured to emit substantially infra-red light and a light receiving element (114a) configured to receive and detect light form the white light emitting element (112a) and the infra-red light emitting element (113a), wherein the white light emitting element (112a) is arranged co-axial and opposite the light receiving element (114a), and wherein the emission axis of the infra-red light emitting element (113a) is arranged substantially orthogonal the incident axis of the light receiving element (114a).

9. The water recycling system according to claim 8, wherein the water quality sensing unit (110) comprises a receptacle (115) for receiving a volume of water and wherein each of the white light emitting element (112a), the infra-red light emitting element (113a), and the light receiving element (114a) are external to and directed into the receptacle (115).

10. The water recycling system according to any of claims 1 to 9, wherein the water collection unit (500) comprises an overflow outlet (504) in fluid connection to and downstream from the outlet (503), the overflow outlet (504) being provided with a siphon (505).

11. The water recycling system according to claim 10, wherein the siphon (505) has a crest height (506) below an upper edge (511) of a sidewall (510) of the water collection unit (500).

12. A method of recycling water in a water recycling system according to any of claims 1 to 11, the method comprising:
- receiving process wash water from a water consumption device (WCD) in the water collection unit (500) via the first inlet (501),
- receiving filtered process wash water from the water recycling device (1) via the separate inlet (502),
- distributing both the process wash water, and the filtered process wash water to the water recycling device (1) via the outlet (503).

## Patentansprüche

1. Wasserrecyclingsystem, umfassend eine Wasserrecyclingvorrichtung (1), wobei die Wasserrecyclingvorrichtung (1) umfasst:
- einen Filtrationstank (100) zum Aufnehmen und Filtern von aufbereitetem Waschwasser,
- einen Verteiler (200), der mit einer Vielzahl von Einlässen (201) zum Aufnehmen von Wasser, einer Vielzahl von Ventilen (202) und einer Vielzahl von Auslässen (203) zum Verteilen von Wasser versehen ist,
- einen Speichertank (300) zum Aufnehmen und Speichern von gefiltertem aufbereitetem Waschwasser aus dem Filtrationstank (100) über einen ersten Auslass (203a) des Verteilers (200),
wobei mindestens ein Einlass (201a) des Verteilers (200) bereitgestellt wird, sodass Wasser aus dem Speichertank (300) in den Verteiler (200) aufgenommen werden kann, und wobei der Verteiler (200) einen zweiten Auslass (203b) zu dem Filtrationstank (100) umfasst, sodass eine Zweiwegeverbindung zwischen dem Filtrationstank (100) und dem Speichertank (300) über den Verteiler (200) bereitgestellt wird, sodass das aufbereitete Waschwasser durch die Wasserrecyclingvorrichtung (1) zirkuliert werden kann, und sodass es mehrere Male gefiltert werden kann, wobei das Wasserrecyclingsystem eine separate Wassersammeleinheit (500) umfasst, wobei die Wassersammeleinheit (500) angeordnet ist, um aufbereitetes Waschwasser von einer Wasserverbrauchsvorrichtung (WCD) über einen ersten Einlass (501) aufzunehmen, und wobei die Wassersammeleinheit (500) mit einem separaten Einlass (502) zum Aufnehmen von gefiltertem aufbereitetem Waschwasser von der Wasserrecyclingvorrichtung (1) und
einem Auslass (503) zum Bereitstellen von Wasser von der Wassersammeleinheit (500) an die Wasserrecyclingvorrichtung (1) versehen ist, sodass sowohl aufbereitetes Waschwasser, das von einer Wasserverbrauchsvorrichtung (WCD) aufgenommen wird, als auch aufbereitetes gefiltertes Waschwasser aus der Wasserrecyclingvorrichtung (1) von der Wassersammeleinheit (500) über den Auslass (503) an die Wasserrecyclingvorrichtung (1) verteilt werden kann.

2. Wasserrecyclingsystem nach Anspruch 1, wobei der Verteiler (200) der Wasserrecyclingvorrichtung (1) einen Auslass (203c) für die Ausgabe von aufbereitetem und gefiltertem Wasser an eine
Wasserverbrauchsvorrichtung (WCD) umfasst.

3. Wasserrecyclingsystem nach Anspruch 1 oder 2, wobei die Wasserrecyclingvorrichtung (1) eine Pumpe (400) in Verbindung mit dem Verteiler (200) für die Verteilung von Wasser umfasst.

4. Wasserrecyclingsystem nach einem der Ansprüche 1 bis 3, wobei der Verteiler (200) der Wasserrecyclingvorrichtung (1) eine einzelne Einheit ist, die interne Kanäle umfasst, die die Vielzahl von Einlässen (201) mit der Vielzahl von Ventilen (202) und Auslässen (203) verbinden.

5. Wasserrecyclingsystem nach einem der Ansprüche 1 bis 4, wobei die Wasserrecyclingvorrichtung (1) eine Wasserqualitätserfassungseinheit (110) umfasst, die mindestens eine Wasserqualitätserfassungsvorrichtung (111) umfasst, und wobei ein Einlass (117) zu der
Wasserqualitätserfassungseinheit (110) stromabwärts des Filtrationstanks (100) angeordnet ist, sodass die Wasserqualitätserfassungseinheit (110) gefiltertes aufbereitetes Waschwasser aufnimmt, und wobei ein Auslass (118) der Wasserqualitätserfassungseinheit (110) angeordnet ist, um Wasser an einen filterseitigen Abschnitt (101) des Filtrationstanks (100) bereitzustellen.

6. Wasserrecyclingsystem nach Anspruch 5, wobei die Wasserrecyclingvorrichtung (1) konfiguriert ist, um das Wasser in dem Speichertank (300) zu der Wasserqualitätserfassungseinheit (110) und danach zu dem filterseitigen Abschnitt (101) des Filtrationstanks (100) zu leiten.

7. Wasserrecyclingsystem nach einem der Ansprüche 5 oder 6, wobei die Wasserrecyclingvorrichtung (1) konfiguriert ist, um dem Speichertank (300) nicht aufbereitetes Leitungswasser über einen Einlass (301) an einem oberen Abschnitt des Speichertanks (300) bereitzustellen.

8. Wasserrecyclingsystem nach einem der Ansprüche 5 bis 7, wobei die Wasserqualitätserfassungsvorrichtung (111) ein Weißlicht emittierendes Element (112a), das konfiguriert ist, um im Wesentlichen weißes Licht zu emittieren, ein Infrarotlicht emittierendes Element (113a), das konfiguriert ist, um im Wesentlichen Infrarotlicht zu emittieren, und ein Licht empfangendes Element (114a) umfasst, das konfiguriert ist, um Licht von dem Weißlicht emittierenden Element (112a) und dem Infrarotlicht emittierenden Element (113a) zu empfangen und zu erkennen, wobei das Weißlicht emittierende Element (112a) koaxial und gegenüber dem Licht empfangenden Element (114a) angeordnet ist, und wobei die Emissionsachse des Infrarotlicht emittierenden Elements (113a) im Wesentlichen orthogonal zur Einfallsachse des Licht empfangenden Elements (114a) angeordnet ist.

9. Wasserrecyclingsystem nach Anspruch 8, wobei die Wasserqualitätserfassungseinheit (110) einen Behälter (115) zum Aufnehmen eines Wasservolumens umfasst und wobei jedes von dem Weißlicht emittierenden Element (112a), dem Infrarotlicht emittierenden Element (113a) und dem Licht empfangenden Element (114a) außerhalb des Behälters (115) angeordnet und in diesen hinein gerichtet ist.

10. Wasserrecyclingsystem nach einem der Ansprüche 1 bis 9, wobei die Wassersammeleinheit (500) einen Überlaufauslass (504) in Fluidverbindung zu und stromabwärts von dem Auslass (503) umfasst, wobei der Überlaufauslass (504) mit einem Siphon (505) versehen ist.

11. Wasserrecyclingsystem nach Anspruch 10, wobei der Siphon (505) eine Scheitelhöhe (506) unterhalb einer Oberkante (511) einer Seitenwand (510) der Wassersammeleinheit (500) aufweist.

12. Verfahren zum Recyceln von Wasser in einem Wasserrecyclingsystem nach einem der Ansprüche 1 bis 11, wobei das Verfahren umfasst:
- Aufnehmen von Prozesswaschwasser von einer Wasserverbrauchsvorrichtung (WCD) in der Wassersammeleinheit (500) über den ersten Einlass (501),
- Aufnehmen von gefiltertem Prozesswaschwasser von der Wasserrecyclingvorrichtung (1) über den separaten Einlass (502),
- Verteilen sowohl des Prozesswaschwassers als auch des gefilterten Prozesswaschwassers an die Wasserrecyclingvorrichtung (1) über den Auslass (503).

## Revendications

1. Système de recyclage d'eau comprenant un dispositif de recyclage d'eau (1), le dispositif de recyclage d'eau (1) comprenant :
- une cuve de filtration (100) destinée à recevoir et filtrer de l'eau de lavage traitée,
- un collecteur (200) pourvu d'une pluralité d'orifices d'entrée (201) destinés à recevoir de l'eau, d'une pluralité de vannes (202) et d'une pluralité d'orifices de sortie (203) destinés à distribuer de l'eau,
- une cuve de stockage (300) destinée à recevoir et stocker de l'eau de lavage traitée filtrée provenant de la cuve de filtration (100) par l'intermédiaire d'un premier orifice de sortie (203a) du collecteur (200),
dans lequel, au moins un orifice d'entrée (201a) du collecteur (200) est prévu de telle sorte que de l'eau peut être reçue en provenance de la cuve de stockage (300) dans le collecteur (200), et dans lequel le collecteur (200) comprend un second orifice de sortie (203b) vers la cuve de filtration (100), de telle sorte qu'un raccordement bidirectionnel entre la cuve de filtration (100) et la cuve de stockage (300) par l'intermédiaire du collecteur (200) est prévu, de telle sorte que l'eau de lavage traitée peut circuler à travers le dispositif de recyclage d'eau (1), et de telle sorte qu'elle peut être filtrée plusieurs fois, dans lequel le système de recyclage d'eau comprend une unité de collecte d'eau (500) séparée, l'unité de collecte d'eau (500) étant agencée pour recevoir l'eau de lavage traitée provenant d'un dispositif de consommation d'eau (WCD) par l'intermédiaire d'un premier orifice d'entrée (501), et dans lequel l'unité de collecte d'eau (500) est pourvue d'un orifice d'entrée séparé (502) destiné à recevoir de l'eau de lavage traitée filtrée provenant du dispositif de recyclage d'eau (1), et
un orifice de sortie (503) destiné à fournir de l'eau à partir de l'unité de collecte d'eau (500) au dispositif de recyclage d'eau (1) de telle sorte que l'eau de lavage traitée reçue en provenance d'un dispositif de consommation d'eau (WCD) et l'eau de lavage filtrée traitée en provenance du dispositif de recyclage d'eau (1) peuvent toutes deux être distribuées à partir de l'unité de collecte d'eau (500), par l'intermédiaire de l'orifice de sortie (503), vers le dispositif de recyclage d'eau (1).

2. Système de recyclage d'eau selon la revendication 1, dans lequel le collecteur (200) du dispositif de recyclage d'eau (1) comprend un orifice de sortie (203c) pour la sortie d'eau traitée et filtrée vers un dispositif de consommation d'eau (WCD).

3. Système de recyclage d'eau selon la revendication 1 ou 2, dans lequel le dispositif de recyclage d'eau (1) comprend une pompe (400) raccordée avec le collecteur (200) pour la distribution d'eau.

4. Système de recyclage d'eau selon l'une quelconque des revendications 1 à 3, dans lequel le collecteur (200) du dispositif de recyclage d'eau (1) est une unité unique comprenant des canaux internes raccordant la pluralité d'orifices d'entrée (201) à la pluralité de vannes (202) et d'orifices de sortie (203).

5. Système de recyclage d'eau selon l'une quelconque des revendications 1 à 4, dans lequel le dispositif de recyclage d'eau (1) comprend une unité de détection de qualité d'eau (110) comprenant au moins un dispositif de détection de qualité d'eau (111), et dans lequel un orifice d'entrée (117) vers l'unité de détection de qualité d'eau (110) est agencé en aval de la cuve de filtration (100) de telle sorte que l'unité de détection de qualité d'eau (110) reçoit de l'eau de lavage traitée filtrée, et dans lequel un orifice de sortie (118) de l'unité de détection de qualité d'eau (110) est agencé pour fournir de l'eau à une partie côté filtre (101) de la cuve de filtration (100).

6. Système de recyclage d'eau selon la revendication 5, dans lequel le dispositif de recyclage d'eau (1) est conçu pour diriger l'eau dans la cuve de stockage (300) vers l'unité de détection de qualité d'eau (110), et ensuite vers la partie côté filtre (101) de la cuve de filtration (100).

7. Système de recyclage d'eau selon l'une quelconque des revendications 5 ou 6, dans lequel le dispositif de recyclage d'eau (1) est conçu pour fournir de l'eau du robinet non traitée à la cuve de stockage (300) par l'intermédiaire d'un orifice d'entrée (301)au niveau d'une partie supérieure de la cuve de stockage (300).

8. Système de recyclage d'eau selon l'une quelconque des revendications 5 à 7, dans lequel le dispositif de détection de qualité d'eau (111) comprend un élément émetteur de lumière blanche (112a) configuré pour émettre une lumière sensiblement blanche, un élément émetteur de lumière infrarouge (113a) configuré pour émettre une lumière sensiblement infrarouge et un élément récepteur de lumière (114a) configuré pour recevoir et détecter de la lumière de l'élément émetteur de lumière blanche (112a) et l'élément émetteur de lumière infrarouge (113a), dans lequel l'élément émetteur de lumière blanche (112a) est agencé coaxial et opposé à l'élément récepteur de lumière (114a), et dans lequel l'axe d'émission de l'élément émetteur de lumière infrarouge (113a) est agencé sensiblement orthogonal à l'axe d'incidence de l'élément récepteur de lumière (114a).

9. Système de recyclage d'eau selon la revendication 8, dans lequel l'unité de détection de qualité d'eau (110) comprend un réceptacle (115) destiné à recevoir un volume d'eau et dans lequel chacun parmi l'élément émetteur de lumière blanche (112a), l'élément émetteur de lumière infrarouge (113a) et l'élément récepteur de lumière (114a) est externe au réceptacle (115) et dirigé vers celui-ci.

10. Système de recyclage d'eau selon l'une quelconque des revendications 1 à 9, dans lequel l'unité de collecte d'eau (500) comprend un orifice de trop-plein (504) en raccordement fluidique à l'orifice de sortie (503) et en aval de celui-ci, l'orifice de trop-plein (504) étant pourvu d'un siphon (505).

11. Système de recyclage d'eau selon la revendication 10, dans lequel le siphon (505) a une hauteur de crête (506) sous un bord supérieur (511) d'une paroi latérale (510) de l'unité de collecte d'eau (500).

12. Procédé de recyclage d'eau dans un système de recyclage d'eau selon l'une quelconque des revendications 1 à 11, le procédé comprenant :
- la réception d'eau de lavage de traitement provenant d'un dispositif de consommation d'eau (WCD) dans l'unité de collecte d'eau (500) par l'intermédiaire du premier orifice d'entrée (501),
- la réception de l'eau de lavage de traitement filtrée provenant du dispositif de recyclage d'eau (1) par l'intermédiaire de l'orifice d'entrée séparé (502),
- la distribution à la fois de l'eau de lavage de traitement et de l'eau de lavage de traitement filtrée au dispositif de recyclage d'eau (1) par l'intermédiaire de l'orifice de sortie (503).
